# FASCICULE DE BREVET EUROPEEN

(11) **EP 2 152 299 B1**
(45) Date de publication et mention de la délivrance du brevet: **25.06.2014**
(21) Numéro de dépôt: 08805491.1
(22) Date de dépôt: 23.04.2008
(51) Int. Cl.: A61K 38/08, A61K 8/64, A61Q 17/04, A61Q 19/08, A61K 38/51, A61P 17/00, A61Q 19/00, C07K 7/00

(54) **COMPOSITION PHARMACEUTIQUE ET/OU COSMETIQUE CONTENANT DES PEPTIDES ACTIVATEURS DE L'ACONITASE**
PHARMAZEUTISCHE UND/ODER KOSMETISCHE ZUSAMMENSETZUNG MIT ACONITASE-AKTIVIERENDEN WIRKSTOFFEN
PHARMACEUTICAL AND/OR COSMETIC COMPOSITION CONTAINING ACONITASE-ACTIVATING ACTIVE INGREDIENTS

(30) Priorité: 27.04.2007 FR 0703053
(43) Date de publication de la demande: 17.02.2010
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2008/000572
(87) Numéro de publication internationale: WO 2008/145849

(56) Documents cités:
- WO-A-95/20046
- WO-A-2004/043482
- WO-A-2006/029028
- FR-A1- 2 893 502
- FR-A1- 2 905 859
- JP-A- 7 233 086
- US-A1- 2004 033 538
- RZYMKIEWICZ D. ET AL.: "Induction of heme oxygenase-1 modulates cis-aconitase activity in lens epitelial cells." BIOCHEM BIOPHYS RES COMM, vol. 270, 2 avril 2000 (2000-04-02), pages 324-328, XP002461446

## Description

La présente invention se situe dans le domaine cosmétique et pharmaceutique, et plus particulièrement dans le domaine de la dermatologie. La présente invention concerne une composition cosmétique ou pharmaceutique, et notamment dermatologique, comprenant, dans un milieu physiologiquement adapté, des principes actifs capables d'activer l'aconitase. Ces principes actifs peuvent être des polypeptides ou des peptides, utilisés seuls ou en association avec au moins un autre principe actif. L'invention est également relative à l'utilisation d'une composition capable de protéger les mitochondries. L'invention porte encore sur un procédé de traitement cosmétique destiné à protéger la peau et les phanères des agressions extérieures et à lutter contre le vieillissement cutané. Lesdits peptides, protecteurs des mitochondries, peuvent également être utilisés pour préparer des compositions pharmaceutiques destinées à prévenir ou lutter contre les pathologies liées à des dysfonctionnements mitochondriaux ; par exemple certaines dégénérescences neuromusculaires ou cardiaques, le diabète de type II ou encore certaines pathologies du vieillissement.

Le terme « phanères » selon l'invention englobe l'ensemble des annexes kératiniques présentes à la surface du corps, en particulier les poils, les cils, les sourcils, les ongles et les cheveux.

Les mitochondries sont les organites des cellules eucaryotes spécialisées dans la production d'énergie. Elles sont dotées de leur propre ADN (ADNmt). L'énergie produite par la chaîne respiratoire est stockée sous forme d'ATP. Dans certaines conditions de stress cellulaire, les électrons, libérés par la chaîne respiratoire de phosphorylation oxydative, produisent des radicaux libres qui peuvent endommager les structures mitochondriales. Ces facteurs de stress peuvent être intrinsèques ou d'origine extérieure. Parmi ces derniers, on peut citer : les rayonnements UV, les toxines, les radiations, les oxydants alimentaires. Ainsi, on a pu montrer un déclin progressif des fonctions mitochondriales avec l'âge, probablement lié à l'accumulation de mutations sur l'ADNmt (K. Singh, Ann. N.Y. Acad. Sci. 1019, 2004). Ces mutations de l'ADNmt peuvent être induites par des expositions répétées aux rayonnements UV, et seraient même un témoin du photo-vieillissement (M. Berneburg et al., J. of Invest. Dermatol. 122(5), 2004).

Les mitochondries jouent également un rôle central dans l'apoptose. Les dégradations mitochondriales peuvent ainsi être un facteur déclenchant de la cascade réactionnelle conduisant à l'apoptose dans de nombreux types cellulaires (G. Kroemer et al., FASEB J. 9:1277-87, 1995).

Dans les mitochondries, des mécanismes naturels de réparations ont été identifiés. L'aconitase (aconitate hydratase ou Iron Regulatory Protein ; EC 4.2.1.3) s'est ainsi révélée être indispensable au maintien de l'intégrité de l'ADNmt, indépendamment de son activité catalytique. L'aconitase a tout d'abord été identifiée comme une enzyme mitochondriale impliquée dans le cycle de Krebs ; elle catalyse la conversion stéréospécifique du citrate en isocitrate et participe ainsi à la production d'énergie de la cellule. Elle possède un site actif comportant un agrégat fer-soufre (4Fe-4S) à géométrie pseudocubique. Récemment l'équipe du D^{r} Butow (X. J. Chen et al., Science 307, 2005) a pu montrer que l'aconitase jouait un rôle clé dans la préservation de l'intégrité de l'ADN mitochondrial. Elle interagirait avec l'ADN à l'intérieur de régions régulatrices suggérant un rôle dans le compactage du génome mitochondrial à la manière d'une protéine de la famille de HSP 70. Le passage de la forme liant l'ADN à la forme active de l'enzyme serait basé sur l'assemblage ou le désassemblage du cluster fer-soufre.

Grâce à sa double fonctionnalité, l'aconitase établit un lien direct entre l'activité génératrice d'énergie de la mitochondrie et la conservation de l'ADNmt. L'aconitase pourrait ainsi constituer une cible thérapeutique pour prévenir ou lutter contre les pathologies liées à des dysfonctionnements mitochondriaux, par exemple, le diabète de type II, certaines affections neuromusculaires, certaines pathologies du vieillissement.

La recherche de composés pouvant stimuler ou protéger les mitochondries est une préoccupation de la recherche médicale et de la cosmétique. Concernant la peau, ces nouveaux composés pourraient être utiles pour prévenir ou lutter contre les signes du vieillissement cutané, mais aussi pour protéger ou réparer la peau après des brûlures, des expositions à des rayonnements UV ou des radiations, ou des expositions à des toxines ou à des polluants.

Afin de protéger les mitochondries et de lutter contre les pathologies associées ou les effets du vieillissement cutané, il a été proposé des solutions telles que l'apport de substances impliquées dans le métabolisme énergétique, et plus particulièrement d'intermédiaires ou de cofacteurs du cycles de Krebs tels que le fumarate, le L-malate, l'acétylCoA (WO 02064129) ou encore le traitement de la peau par des substances capables de diminuer les radicaux libres, telles que la vitamine C (US 2004/0086526) ou la L-ergothionéine (WO 9836748). Mais, à la connaissance de la demanderesse, une composition contenant des principes actifs capables d'activer l'aconitase et son usage, dans le but de protéger les mitochondries et de lutter contre les pathologies associées et/ou les effets du vieillissement cutané, n'a jamais été décrit.

La présente invention a pour principal objectif de fournir un nouveau principe actif capable de protéger la peau des agressions extérieures et de lutter contre le vieillissement cutané. Les inventeurs ont en effet mis en évidence une activité thérapeutique, et plus particulièrement dermatologique et cosmétique, de peptides capables d'activer l'aconitase. Il a notamment été mis en évidence que ces peptides, lorsqu'ils sont appliqués sur la peau, favorisent de façon importante l'activité mitochondriale, démontrée par une activité accrue de l'enzyme aconitase et une augmentation de la synthèse d'ATP. Ces nouveaux principes actifs protecteurs des mitochondries permettent ainsi d'ouvrir de nouvelles perspectives thérapeutiques et cosmétiques.

On entend par « principe actif protecteur des mitochondries ou capable de protéger les mitochondries » toute substance capable de limiter les altérations fonctionnelles ou structurales des mitochondries de cellules ou de tissus soumis à un stress physico-chimique ou environnemental.

On entend par « peptide capable d'activer l'aconitase » tout peptide ou polypeptide capable d'augmenter l'aconitase, cytoplasmique et/ou mitochondriale, soit par l'activation de la synthèse protéique de l'aconitase (par modulation directe ou indirecte de l'expression génique de l'aconitase), soit par l'augmentation de l'activité enzymatique de l'aconitase, soit par d'autres processus biologiques tels que la stabilisation de la protéine aconitase ou encore la stabilisation des transcrits d'ARN messager.

Le terme « peptide » désigne un enchaînement de deux ou plusieurs acides aminés liés entre eux par des liaisons peptidiques ou par des liaisons peptidiques modifiées ; le terme « polypeptide » désignant un peptide de taille plus importante.

Par « peptide », il faut entendre le peptide naturel ou synthétique de l'invention tel que décrit ci-dessus ou au moins l'un de ses fragments, qu'il soit obtenu par protéolyse ou de manière synthétique, ou encore tout peptide naturel ou synthétique dont la séquence est totalement ou partiellement constituée par la séquence du peptide selon l'invention.

Ainsi, l'invention a pour premier objet une composition cosmétique ou pharmaceutique contenant, dans un milieu physiologiquement acceptable, en tant que principe actif protecteur des mitochondries, un peptide capable d'activer l'aconitase, dont le nombre d'acides aminés est compris entre 3 et 14, et qui possède une séquence qui répond à la formule générale (I) :

R₁-(AA)ₙ- X₁-X₂- X₃- X₄-X₅- X₆-(AA)ₚ-R₂

Dans laquelle
X₁ est la serine,
X₂ est la cystéine,
X₃ est la thréonine ou l'isoleucine,
X₄ est la glutamine, l'asparagine ou aucun acide aminé,
X₅ est la thréonine, la sérine ou aucun acide aminé,
X₆ est l'isoleucine, la glutamine ou aucun acide aminé,
AA représente un acide aminé quelconque ou un de ses dérivés, absent de la séquence de l'aconitase, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄, correspondant à une des séquences suivantes :
(SEQ ID n° 1) Ser-Cys-Ile-Asn-Thr
(SEQ ID n°2) Ser-Cys-Ile-Asn-Thr-NH₂
(SEQ ID n°5) Ser-Cys-Ile-Asn-Ser

Selon un mode de réalisation particulièrement intéressant, le peptide biologiquement actif correspond à la séquence SEQ ID n°1.

Selon un autre mode de réalisation particulièrement intéressant, le peptide biologiquement actif correspond à la séquence SEQ ID n°2.

Dans l'invention, le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une composition telle que définie précédemment, caractérisée par le fait que le peptide de séquence SEQ ID n°1, SED ID n°2 et SEQ ID n°5 est sous forme protégée ou non. On peut utiliser une protection basée sur une substitution sur l'extrémité amino-terminale par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. D est également possible de protéger les deux extrémités du peptide.

Les dérivés de peptides concernent aussi les acides aminés et les peptides reliés entre eux par une liaison pseudo-peptidique. On entend par « liaison pseudo-peptidique » tous les types de liaisons susceptibles de remplacer les liaisons peptidiques « classiques ».

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration Let D- ; de manière préférentielle, les acides aminés sont sous forme L. Le peptide selon l'invention peut donc être sous forme L-, D- ou DL-.

Le peptide de formule générale (I) de séquence SEQ ID n°1, SEQ ID n°2 et SEQ ID n°5 selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut également être obtenu par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement aux peptides de formule générale (I) de séquence SEQ ID n°1, SEQ ID n°2 et SEQ ID n°5.

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides de formule générale I de séquence SEQ ID n°1, SEQ ID n°2 et SEQ ID n°5 selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi le peptide selon l'invention peut être d'origine naturelle ou synthétique. Préférentiellement selon l'invention, le peptide est obtenu par synthèse chimique.

Selon l'invention, le principe actif peut être un mélange de dérivés peptidiques et/ou constitués de dérivés d'acides aminés.

Selon un mode de réalisation avantageux de l'invention, les polypeptides ou peptides de formule générale (I) de séquence SEQ ID n°1, SEQ ID n°2 et SEQ ID n°5 sont préalablement solubilisés dans un ou plusieurs solvants cosmétiquement ou pharmaceutiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les peptides possédant la formule générale (I) de séquence SEQ ID n°1, SEQ ID n°2 et SEQ ID n°5 selon l'invention sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu que le peptide selon l'invention peut être utilisé seul ou bien en association avec au moins un autre principe actif, dans une composition cosmétique ou pour la préparation d'une composition pharmaceutique et/ou dermatologique.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, d'émulsions huile-dans-eau ou eau-dans-huile, d'émulsions multiples, de solutions, de suspensions, de gels, de laits, de lotions, de sticks ou encore de poudres, adaptés à une application sur la peau, les lèvres et/ou les phanères.

Ces compositions comprennent les excipients nécessaires à leur formulation, tels que solvants, épaississants, diluants, tensioactifs, antioxydants, colorants, conservateurs, parfums.

Bien entendu, l'homme de métier veillera à choisir les éventuels composés complémentaires, actifs ou non-actifs, et/ou leur quantité, de telle sorte que les propriétés avantageuses du mélange ne soient pas, ou sensiblement pas, altérées par l'adjonction envisagée.

La composition utilisable selon l'invention peut en particulier consister en une composition pour soins capillaires, et notamment un shampooing, un après-shampooing, une lotion de mise en plis, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non suivie d'un rinçage, ou encore sous forme de shampooing.

Elle peut également se présenter sous forme de teinture ou de mascara à appliquer au pinceau ou au peigne, en particulier sur les cils, les sourcils ou les cheveux.

Avantageusement, les compositions utilisables selon l'invention contiennent en outre d'autres principes actifs favorisant l'action du principe actif selon l'invention. Par exemple, il peut être ajouté des principes actifs ayant une action anti-radicalaire ou antioxydante, choisis parmi la vitamine C, la vitamine E ou le coenzyme Q10 ou les extraits polyphénoliques de plantes.

Par « principes actifs anti-radicalaires », on entend tout composé capable de piéger les radicaux libres. Ces principes actifs sont capables de bloquer les réactions en chaînes des radicaux libres avant les étapes ultimes de dégradation des constituants biologiques de la peau et ont de ce fait une activité antioxydante.

La composition selon l'invention peut encore associer aux peptides activateurs de l'aconitase, des principes actifs stimulant les synthèses des macromolécules dermiques (laminine, fibronectine, collagène), par exemple le peptide de collagène commercialisé sous le nom « Collaxyl® » par la société Vincience.

La composition selon l'invention peut également associer aux peptides activateurs de l'aconitase des principes actifs stimulant le métabolisme énergétique, comme le principe actif commercialisé sous la dénomination « GP4G® » par la société Vincience.

Selon un autre aspect, la composition selon l'invention peut être une composition solaire, c'est-à-dire une composition aidant à la protection contre le rayonnement solaire. Ainsi, il peut être avantageusement ajouté, à la composition selon l'invention, des actifs aidant à la protection solaire tel que, par exemple, des filtres solaires.

D est bien évident que l'invention s'adresse aux mammifères en général et plus particulièrement aux êtres humains.

La quantité efficace de principe actif selon l'invention correspond à la quantité nécessaire afin d'obtenir le résultat recherché, à savoir ; activer l'aconitase, protéger les mitochondries et ainsi protéger la peau des agressions extérieures et lutter contre le vieillissement cutané.

Selon un mode de réalisation avantageux de l'invention, le peptide de formule générale (I) de séquence SEQ ID n°1, SEQ ID n°2 et SEQ ID n°5 est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse.

Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des antioxydants, des colorants, des filtres solaires, des principes auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Par ses activités particulières, le peptide selon l'invention pourra être utilisé avantageusement dans une composition cosmétique ou pour la préparation d'une composition pharmaceutique.

En particulier, le peptide selon l'invention pourra être utilisé avantageusement dans une composition cosmétique destinée à lutter de manière préventive et/ou curative contre les manifestations du vieillissement cutané et, plus spécifiquement, afin de lutter contre et/ou de prévenir le vieillissement photo-induit (photo-vieillissement). Par manifestations cutanées du vieillissement, on entend toutes modifications de l'aspect extérieur de là peau dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, mais également toute modification interne de la peau qui ne se traduit pas systématiquement par un aspect extérieur modifié comme, par exemple, toute dégradation interne de la peau consécutive à une exposition aux rayonnements ultraviolets (UV). Le peptide selon l'invention, ou la composition le contenant, permettra de lutter, en particulier, contre la perte d'élasticité et de fermeté de la peau.

Le peptide selon l'invention permet de protéger la peau et les phanères contre tous types d'agressions extérieures. L'utilisation du peptide, ou d'une composition le contenant, va permettre à la peau et aux phanères d'être protégés et de mieux résister aux stress environnementaux.

On entend, par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure », due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette.

Le peptide selon l'invention peut être avantageusement utilisé dans une composition cosmétique ou pour la préparation d'une composition pharmaceutique, en tant que principe actif photo-protecteur et, plus particulièrement, en tant que principe actif photo-protecteur dit « secondaire ». On distingue, en effet, les principes actifs photo-protecteurs primaires des principes actifs photo-protecteurs secondaires. Les principes actifs photo-protecteurs primaires sont des substances qui exercent un pouvoir physique : ils sont en mesure d'absorber les rayonnements UV et de les restituer sous forme de chaleur afin de protéger la peau. Les principes actifs photo-protecteurs secondaires sont des substances qui ont généralement un effet biologique ; ce sont, par exemple, les principes actifs capables de limiter les dommages causés à l'ADN et aux membranes par la pénétration des rayonnements UV dans la peau.

Ainsi, l'invention a également pour objet l'utilisation dans une composition cosmétique, ou pour la préparation d'une composition pharmaceutique, d'une quantité efficace de peptide tel que décrit précédemment, le peptide, ou la composition le contenant, étant destiné à protéger la peau des expositions aux UV ou aux rayonnements ionisants lors de radiothérapies.

L'invention a également pour objet l'utilisation dans une composition cosmétique, ou pour la préparation d'une composition pharmaceutique, d'une quantité efficace de peptide selon l'invention, le peptide, ou la composition le contenant, étant destiné à protéger les mitochondries et à augmenter la synthèse d'ATP intracellulaire des cellules de la peau.

L'invention se rapporte encore à l'utilisation dans une composition cosmétique, ou pour la préparation d'une composition pharmaceutique, d'une quantité efficace de peptide tel que décrit précédemment, le peptide, ou la composition le contenant, étant destiné à protéger la peau des dommages causés par les radicaux libres.

L'invention consiste encore en l'utilisation du principe actif selon l'invention pour préparer une composition pharmaceutique destinée à prévenir ou lutter contre les dysfonctionnements mitochondriaux responsables de certaines dégénérescences neuromusculaires ou cardiaques, le diabète de type II, ou encore certaines pathologies du vieillissement.

L'invention consiste encore en un procédé de traitement cosmétique destiné à protéger la peau et les phanères des agressions extérieures et à lutter contre le vieillissement cutané, caractérisé par l'application sur la peau ou les phanères à traiter d'une composition contenant une quantité efficace de l'agent actif selon l'invention.

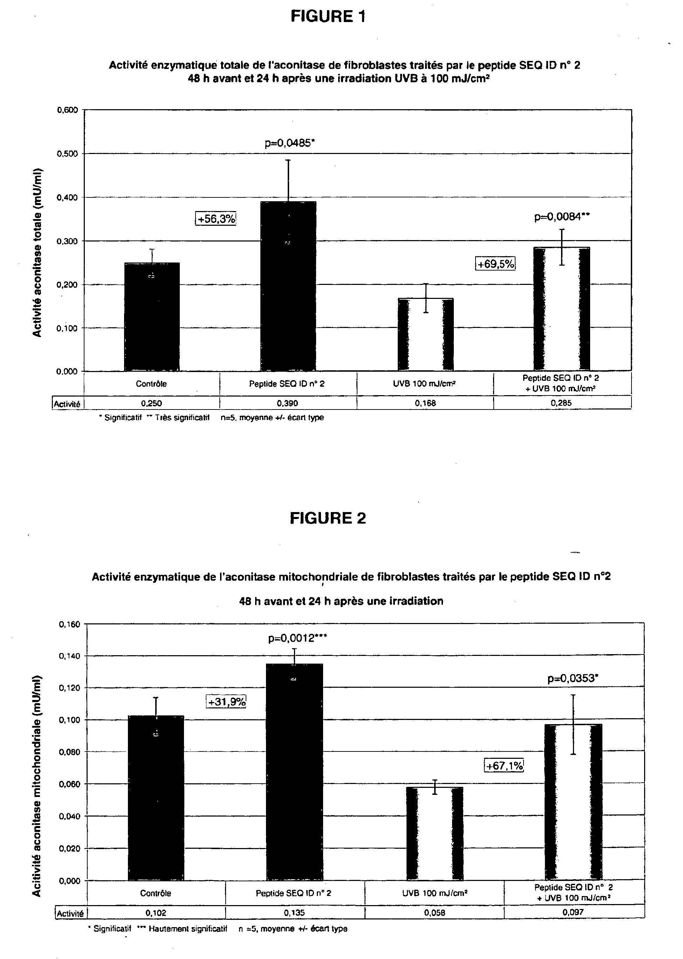

Des modes particuliers de réalisation de ce procédé de traitement cosmétique résultent également de la description précédente. D'autres avantages et caractéristiques de l'invention apparaîtront mieux à la lecture des exemples donnés à titre illustratif et non limitatif.
**La** **figure 1** représente les résultats du dosage de l'activité enzymatique totale de l'aconitase dans des fibroblastes traités par le peptide SEQ ID n°2 et irradiés ou non par des UVB.
**La** **figure 2** représente les résultats du dosage de l'activité enzymatique spécifique de l'aconitase mitochondriale dans des fibroblastes traités par le peptide SEQ ID n°2 et irradiés ou non par des UVB.

### Exemple 1: Mise en évidence de l'effet stimulant du peptide SEQ ID_n°1 sur la synthèse d'ATP intracellulaire

Le but de cette étude est de déterminer l'influence du peptide SEQ ID n°1 sur l'activité mitochondriale. C'est l'ATP, produit par les mitochondries, qui sert de marqueur biochimique pour cette activité.

Protocole : Cette étude s'effectue à l'aide d'un Kit « ATP Bioluminescence Assay Kit HS II » (Roche Applied Science). Les fibroblastes dermiques sont traités avec une solution à 1 %, d'une solution à 50 ppm, contenant le peptide SEQ ID n°1, représentatif de la famille de peptide selon l'invention, pendant une période allant de 1 à 3 heures. A la fin des temps d'incubation, les puits sont vidés de leur milieu et rincés avec 2 ml de PBS froid avant d'ajouter 250 µl d'un tampon de lyse fournis par le kit. Les cellules de chaque puits sont ensuite grattées puis récoltées dans des tubes de 14 ml. Chaque puits est rincé avec 2 x 500 µl PBS froid et le tout est de nouveau récolté dans les tubes respectifs. A partir de ces échantillons, une dilution est réalisée au 1/12000^{ième} dans du PBS froid avant chaque lecture. Le dosage d'ATP est réalisé sur ces échantillons : 50 µL de cette dilution sont déposés dans une luma-cuvette et 50 µL de luminol sont ajoutés. Après 10 secondes, la lecture de la luminescence est déclenchée. Les valeurs sont standardisées par rapport à la quantité de protéines pour chaque échantillon. Les mesures sont effectuées à l'aide d'un appareil : le Biocounter M2010A LUMAC®/3M.

Résultats : Les dosages d'ATP montrent qu'il y a une nette augmentation de la quantité d'ATP intracellulaire après 1 heure (+ 43 %) et après 3 heures, dans des cellules traitées par le peptide SEQ ID n°1, en comparaison des cellules non traitées.

Conclusion : Le peptide SEQ ID n°1 stimule très significativement l'activité mitochondriale de cellules cutanées telles que les fibroblastes.

### Exemple 2: Mise en évidence de l'effet activateur du peptide SEQ ID n°2 sur l'activité enzymatique de l'aconitase

Le but de cette étude est de déterminer l'influence du peptide SEQ ID n°2 sur l'activité enzymatique de l'aconitase. Pour cela l'activité enzymatique totale (cytoplasmique et mitochondriale) et l'activité enzymatique mitochondriale de l'aconitase ont été mesurées.

Protocole : Des fibroblastes humains normaux sont traités avec une solution à 1 %, d'une solution à 50 ppm, contenant le peptide SEQ ID n°2, représentatif de la famille de peptide selon l'invention, pendant 48h, puis soumis à une irradiation par des UVB à 100mJ/cm² . Après l'irradiation, les cellules sont traitées dans les mêmes conditions pendant encore 24h. Des contrôles négatifs sont réalisés dans les mêmes conditions mais en l'absence de peptide SEQ ID n°2.

Pour évaluer l'activité enzymatique de l'aconitase, les fibroblastes sont ensuite récoltés et centrifugés dans du tampon PBS. Une partie des cellules est ensuite traitée pour réaliser le dosage de l'activité enzymatique totale, une autre partie est utilisée pour isoler la fraction mitochondriale selon le protocole suivant :

Les cellules sont lysées à l'aide d'un homogénéisateur de type Dounce et l'homogénat est centrifugé à froid, à 10000 g pendant 10 minutes. Le culot, riche en mitochondries, est lavé avec du tampon TES (10 mM Tris, 1 mM EGTA, 0,25 M sucrose) et resuspendu dans du tampon 0,2 mM citrate trisodique avant d'être utilisé pour le dosage enzymatique.

Le dosage de l'activité enzymatique de l'aconitase est réalisé à l'aide du Kit « OXIS Bioxytech Aconitase-340 assay » (Oxys International), selon le principe suivant : L'aconitase provoque l'isomérisation du citrate en isocitrate. L'isocitrate est ensuite transformé en alpha-ketoglutarate par l'enzyme isocitrate déshydrogénase. cette deuxième réaction s'accompagne de la transformation de NADP+ en NADPH. C'est la formation de NADPH qui est quantifiée par spectrophotométrie à 340 nm. Dans les conditions expérimentales choisies, la vitesse de formation du NADPH est proportionnelle à la quantité d'aconitase. L'analyse de la cinétique d'apparition du NADPH permet de calculer la concentration en aconitase de l'échantillon (en mU/ml).

Résultats : Les résultats proviennent de 5 expériences indépendantes (n=5). Les dosages de l'activité enzymatique totale de l'aconitase (figure 1) montrent une augmentation de 56,3% de l'activité dans les cellules traitées par le peptide SEQ ID n°2 comparées aux cellules contrôle non traitées. Lorsque les cellules sont irradiées par les UVB on observe au contraire une diminution de 33% de cette activité. Le traitement préalable et postérieur à l'irradiation UVB par le peptide de séquence SEQ ID n° 5 permet de limiter la diminution observée dans les cellules contrôle non traitées.

Les dosages de l'activité enzymatique de l'aconitase mitochondriale (figure 2) montrent des résultats comparables.

Les résultats sont présentés dans les figures 1 et 2.

Conclusions : Le peptide SEQ ID n°2, à la concentration de 0,5 ppm, stimule fortement l'activité enzymatique des aconitases cytoplasmiques et mitochondriales des cellules cutanées. L'action du peptide SEQ ID n°2 protège les cellules et les mitochondries vis à vis d'un stress par les UVB.

### Exemple 3 : Evaluation de l'effet protecteur du peptide SEQ ID n°2 sur les cellules cutanées soumises à des rayonnements ultraviolets (UVB)

Le but de cette étude est de déterminer l'effet protecteur du peptide SEQ ID n°2 vis-à-vis de fibroblastes dermiques soumis à un stress par des rayonnements UVB. Pour cela, des tests de viabilité cellulaire ont été réalisés par la technique au MTT.

Protocole : Les fibroblastes dermiques humains sont traités avec une solution à 1 %, d'une solution à 50 ppm de peptide SEQ ID n°2, pendant 24 heures, irradiés par des UVB (de 25 à 100 mJ/cm²) puis cultivés encore 24 heures en présence de la même concentration de peptide SEQ ID n°2. Des contrôles non traités et non irradiés et des contrôles non traités mais irradiés sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure). Ce composé est absorbé par les cellules vivantes puis métabolisé par les enzymes mitochondriales en un composé bleu violet, le formazan, qui sera dosé par spectrophotométrie à 540 nm. La densité optique (D.O.) est alors directement proportionnelle à l'activité enzymatique mitochondriale ainsi qu'au nombre de cellules vivantes.

### Résultats :

| Conditions | Contrôle sans Peptide | Peptide | Contrôle UVB | Peptide + UVB | Contrôle UVB | Peptide + UVB | Contrôle UVB | Peptide + UVB |
|---|---|---|---|---|---|---|---|---|
| Peptide SEQ ID n°2 (ppm) | - | 0,5 | | 0,5 | | 0,5 | | 0,5 |
| Irradiations UVB (mJ/cm²) | - | - | 25 | 25 | 50 | 50 | 100 | 100 |
| % de viabilité | 100 | 114,24 | 94,76 | 108,24 | 88,59 | 102,09 | 76,43 | 98,18 |

L'évaluation de la viabilité cellulaire par la technique du MTT montre d'une part que le peptide SEQ ID N°2 indépendamment de tout rayonnement UVB augmente la viabilité cellulaire et, d'autre part, que les irradiations par les UVB ont eu un effet cytotoxique dose-dépendant sur les fibroblastes humains, mais que cet effet cytotoxique ne se manifeste pas en présence de 0,5 ppm de peptide SEQ ID n°2.

Conclusions : Le peptide SEQ ID n°2, à la concentration de 0,5 ppm, augmente la viabilité cellulaire et, d'autre part, protège efficacement les cellules cutanées contre les effets cytotoxiques des rayonnements UVB.

### Exemple 4 : Evaluation de l'effet protecteur du peptide SEQ ID n°2 sur les cellules cutanées soumises à un stress oxydatif

Le but de cette étude est de déterminer l'effet protecteur du peptide SEQ ID n°2 vis-à-vis de fibroblastes dermiques soumis à un stress oxydatif provoqué par de l'eau oxygénée (H₂O₂) à 4 mM ou 5 mM. Pour cela, des tests de viabilité cellulaire ont été réalisés par la technique au MTT.

Protocole : Les fibroblastes dermiques humains sont traités avec une solution à 1 %, d'une solution à 50 ppm de peptide SEQ ID n°2, pendant 24 heures, soumis à un stress oxydatif provoqué par de l'H₂O₂ à 4 mM ou 5 mM, pendant 30 minutes puis cultivés encore 24 heures en présence de la même concentration de peptide SEQ ID n°2. Des contrôles non traités par le peptide ni par de l'H₂O₂ et des contrôles de traitement avec le peptide seul sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure), selon le protocole décrit dans l'exemple 3.

### Résultats :

| Conditions | Contrôle sans Peptide | Peptide | Contrôle H₂O₂ | Peptide + H₂O₂ | Contrôle H₂O₂ | Peptide + H₂O₂ |
|---|---|---|---|---|---|---|
| Peptide SEQ ID n°2 (ppm) | - | 0,5 | - | 0,5 | - | 0,5 |
| H₂O₂ - 30min | - | - | 4 mM | 4 mM | 5 mM | 5 mM |
| % de viabilité | 100 | 121,13 | 91,71 | 102,04 | 68,92 | 94,40 |

L'évaluation de la viabilité cellulaire par la technique du MTT montre, d'une part, que le peptide SEQ ID N°2, indépendamment d'un stress oxydatif, augmente la viabilité cellulaire et d'autre part, qu'un stress oxydatif provoqué par de l'H₂O₂ à 4 mM ou 5 mM pendant 30 minutes, a eu un effet cytotoxique dose-dépendant sur les fibroblastes humains, mais que cet effet cytotoxique ne se manifeste pas en présence de 0,5 ppm de peptide SEQ ID n°2.

Conclusions : Le peptide SEQ ID n°2, à la concentration de 0,5 ppm, augmente la viabilité cellulaire et protège efficacement les cellules cutanées contre les effets cytotoxiques d'un stress oxydatif.

### Exemple 5: Evaluation de l'effet cytoprotecteur du peptide SEQ ID n°2 sur les cellules cutanées soumises à des rayonnements ultraviolets (UVB)

Une étude de l'effet cytoprotecteur du peptide SEQ ID n°2, a été réalisée en évaluant les dommages provoqués au niveau de l'ADN. Cette étude a été réalisée grâce au test des comètes (ou « Single Cell Gel Electrophoresis »), une technique micro-électrophorétique courte et sensible qui permet de visualiser et de quantifier les cassures de l'ADN sur des cellules individuelles.

Protocole : Des fibroblastes humains primaires sont ensemencés dans des boîtes de diamètres 100. Lorsque les cellules ont atteint 70 % de confluence, elles sont traitées avec le peptide SEQ ID n°2, dilué à 1 % (à partir d'une solution à 50 ppm) pendant 24 heures. Les cellules sont ensuite soumises à une irradiation UVB à 100 mJ/cm², puis cultivées en présence du peptide pendant encore 24 heures. Des boîtes non traitées par le peptide servent de contrôle. Une suspension cellulaire à 100 000 cellules/ml est ensuite réalisée, 500 µl sont prélevés, inclus dans une solution d'agarose et coulés entre lame et lamelle. Les cellules sont lysées à froid. L'ADN est ensuite dénaturé par un tampon alcalin puis soumis à une courte électrophorèse (250 mA pendant 30 minutes) et mis en évidence par une coloration à l'iodure de propidium. Les lames sont alors observées au microscope à fluorescence. Une cellule altérée voit son ADN s'étirer vers l'anode proportionnellement au nombre de cassures présentes dans l'ADN et forme « une comète ». L'ADN fortement dégradé se trouve dans la « queue » de la comète. Une cellule intacte reste ronde et son ADN est alors compacté au niveau de la « tête » de la comète.

L'évaluation des cassures de l'ADN s'effectue à l'aide d'un logiciel analyseur d'image qui permet de déterminer le pourcentage de dégradation de l'ADN, par l'évaluation quantitative du « Tail Moment », un paramètre se définissant comme le produit de la longueur de la comète par le pourcentage d'ADN dans sa partie distale.

Résultats : Le tableau ci-dessous représente l'évaluation des « Tail Moment » mesurés dans des fibroblastes irradiés par les UVB et traités ou non par le peptide SEQ ID n°2.

| **Conditions d'étude** | **Mesure du « Tail Moment »** | **% du « Tail Moment » par rapport au contrôle UV** |
|---|---|---|
| Contrôle UVB | 106563 | 100 |
| UVB + peptide SEQ ID n°2 | 80221 | 75 |

Les résultats obtenus montrent que les cellules soumises à une irradiation UVB, en l'absence du peptide SEQ ID n°2 (contrôle UVB) présentent le « Tail Moment » le plus important, c'est-à-dire la condition où l'ADN est le plus endommagé. Au contraire, les cellules traitées avec le peptide SEQ ID n°2 à 0,5 ppm selon l'invention, ont un «Tail Moment », c'est à dire des dommages à l'ADN, diminué de 25 %.

Conclusion : Le peptide SEQ ID n°2 joue un rôle important dans la protection de l'ADN des cellules cutanées soumises à une irradiation par les UVB.

### Exemple 6 : Evaluation de l'action du peptide SEQ ID n°2 sur les l'ultrastructure des mitochondries

Le but de cette étude est de déterminer l'action du peptide SEQ ID n°2 sur les mitochondries, par l'observation des ultrastructures en microscopie électronique.

Protocole : Des kératinocytes humains normaux sont traités avec une solution à 1 %, d'une solution mère à 50 ppm de peptide SEQ ID n°2, pendant 72 heures. Des contrôles négatifs sont réalisés dans les mêmes conditions mais en l'absence dudit peptide.

Les cellules sont ensuite fixées par le fixateur de Karnosky (20 ml de paraformaldehyde 16%/ 8 ml de glutaraldehyde 50% / 25 ml de tampon sodium phosphate 0,2M / 25 ml d'eau), 1h à température ambiante puis laissées une nuit à 4°C. Les supports de culture sont grattés dans le fixateur de Karnosky puis les cellules sont centrifugées pendant 5 minutes à 5000 rpm. Le surnageant est écarté et le culot resuspendu dans 1 ml de cacodylate à 0,1 M. Les échantillons sont ensuite conservés à 4°c jusqu'à leur traitement pour l'observation en microscopie électronique.

Résultats : Les organites des cellules traitées par le peptide SEQ ID n°2 présentent des caractéristiques ultrastructurales suggérant une augmentation de l'activité métabolique. En particulier, les spécificités de la population de mitochondries, et l'abondance des éléments de l'appareil de Golgi concordent avec une stimulation de la synthèse métabolique.

Conclusion : Le peptide SEQ ID n°2 a une action stimulante sur l'activité mitochondriale.

### Exemple 7 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 1 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2 -Lait après-soleil :

| Noms commerciaux | Noms INCI | % massique |
|---|---|---|
| PHASE A | | |
| Montanov L | C14-22 Alcohols (and) C12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| PHASE B | | |
|---|---|---|
| Eau déminéralisée | Aqua (Water) | qsp |

| PHASE C | | |
|---|---|---|
| Simulgel EG | Sodium Acrylate/Acryloyldimethyl Taurate Copolymer (and) Isohexadecane (and) Polysorbate 80 Copolymer (and) Polysorbate 80 | 0,4 |

| PHASE D | | |
|---|---|---|
| Phenonip | Phenoxyethanol (and) Methylparaben (and) Ethylparaben (and) Butylparaben (and) Propylparaben (and) Isobutylparaben Ethylparaben and Propylparaben and Buthylparaben | 0,30 |
| Germall 115 | Imidazolidinyl Urea | 0,20 |

| PHASE E | | |
|---|---|---|
| Peptide SEQ ID n° 2 | | 0,1 ppm |

Préparer la phase A sous agitation. Incorporer la gomme xanthane progressivement, sous agitation défloculeuse. Les phases C et D seront incorporées une fois le gel terminé. La phase E, préparée préalablement jusqu'à parfaite dissolution de la DHA, sera rajoutée ensuite. Ajuster le pH si nécessaire à 4 - 4,5. Colorer et parfumer.

### 3 -Crème anti-âge :

| ***Noms commerciaux*** | ***Noms INCI*** | ***% massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Noms commerciaux*** | ***Noms INCI*** | ***% massique*** |
|---|---|---|
| ***Phase D*** | | |
| TEA | Triethanolamine | 0,18 |

| ***Phase E*** | | |
|---|---|---|
| Peptide SEQ ID n°1 | | 0,5 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 4 -Crème protectrice de jour :

| ***Noms commerciaux*** | ***Noms INCI*** | ***% massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°2 | | 5 ppm |

Préparer la phase A et chauffer à 75°C sous agitation. Préparer la phase B en dispersant le carbopol, puis la gomme xanthane sous agitation. Laisser reposer. Chauffer à 75°C. A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Composition cosmétique ou pharmaceutique contenant, dans un milieu physiologiquement acceptable, en tant que principe actif protecteur des mitochondries, un peptide capable d'activer l'aconitase, comprenant de 3 à 14 résidus d'acides aminés, et dont la séquence correspond à la formule générale (I) :
R₁-(AA)ₙ-X₁-X₂-X₃-X₄-X₅-X₆-(AA)ₚ-R₂
dans laquelle
X₁ est la serine,
X₂ est la cystéine,
X₃ est la thréonine ou l'isoleucine,
X₄ est la glutamine, l'asparagine ou aucun acide aminé,
X₅ est la thréonine, la serine ou aucun acide aminé,
X₆ est l'isoleucine, la glutamine ou aucun acide aminé,
AA représente un acide aminé quelconque ou un de ses dérivés, absent de la séquence de l'aconitase, et n et p sont des nombres entiers compris entre 0 et 4.
R₁ représente la fonction aminé primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C- terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄,
**caractérisée en ce que** ledit peptide de formule générale (I) est de séquence :
(SEQ ID n°1) Ser-Cys-Ile-Asn-Thr
(SEQ ID n°2) Ser-Cys-Ile-Asn-Thr-NH₂
(SEQ ID n°5) Ser-Cys-Ile-Asn-Ser.

2. Composition selon la revendication 1, **caractérisée en ce que** ledit peptide de formule générale (I) correspond à la séquence SEQ ID n°1.

3. Composition selon la revendication 1, **caractérisée en ce que** ledit peptide de formule générale (I) correspond à la séquence SEQ ID n°2.

4. Composition selon l'une quelconque des revendications précédentes, **caractérisée en ce que** ledit peptide de formule générale (I) possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

5. Composition selon l'une quelconque des revendications précédentes, **caractérisée en ce que** ledit peptide de formule générale (I) est présent dans la composition à une concentration comprise entre 0,0005 et 500 ppm.

6. Composition selon la revendication 5, **caractérisée en ce que** ledit peptide de formule générale (I) est présent dans la composition à une concentration comprise entre 0,01 et 5 ppm.

7. Composition selon l'une quelconque des revendications précédentes, **caractérisée en ce que** ledit peptide de formule générale (I) est préalablement solubilisé dans un ou plusieurs solvants cosmétiquement ou pharmaceutiquement acceptables choisi parmi l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

8. Composition selon l'une quelconque des revendications précédentes, **caractérisée en ce qu'**elle se présente sous une forme adaptée à l'application par voie topique comprenant un milieu cosmétiquement ou dermatologiquement acceptable.

9. Composition selon l'une quelconque des revendications précédentes, **caractérisée en ce qu'**elle contient, en outre, au moins un autre principe actif favorisant l'action dudit peptide de formule générale (I) choisi parmi la vitamine C, la vitamine E, le coenzyme Q10, les extraits polyphénoliques de plantes, le peptide de séquence Gly-Pro-Gln-Gly-Pro-Gln commercialisé sous le nom de COLLAXYL® par Vincience ou l'extrait d'*Artemia* commercialisé sous le nom de GP4G® par Vincience.

10. Principe actif, tel que défini dans l'une quelconque des revendications 1 à 7, pour son utilisation dans une composition destinée à protéger la peau et les phanères contre les rayonnements UV.

11. Principe actif, tel que défini dans l'une quelconque des revendications 1 à 7, pour son utilisation dans une composition destinée à prévenir ou à traiter les dommages causés à la peau et aux phanères par les radicaux libres.

12. Utilisation dans une composition cosmétique, d'une quantité efficace de principe actif, tel que défini dans l'une quelconque des revendications 1 à 7, **caractérisée en ce que** la composition est destinée à prévenir ou à traiter les signes cutanés du vieillissement et/ou du photo-vieillissement.

13. Principe actif, tel que défini dans l'une quelconque des revendications 1 à 7, pour son utilisation dans une composition pharmaceutique destinée à prévenir ou à lutter contre les dysfonctionnements mitochondriaux responsables de certaines dégénérescences neuromusculaires ou cardiaques, du diabète de type II ou encore de certaines pathologies du vieillissement.

## Patentansprüche

1. Kosmetische oder pharmazeutische Zusammensetzung, die in einem physiologisch unbedenklichen Medium als Wirkstoff zum Schutz der Mitochondrien ein Peptid enthält, das Aconitase aktivieren kann, 3 bis 14 Aminosäurereste umfasst und dessen Sequenz der allgemeinen Formel (I) entspricht:
R₁-(AA)ₙ-X₁-X₂-X₃-X₄-X₅-X₆-(AA)ₚ-R₂,
wobei
X₁ Serin ist,
X₂ Cystein ist,
X₃ Threonin oder Isoleucin ist,
X₄ Glutamin, Asparagin oder keine Aminosäure ist,
X₅ Threonin, Serin oder keine Aminosäure ist,
X₆ Isoleucin, Glutamin oder keine Aminosäure ist,
AA für eine beliebige Aminosäure oder eines ihrer Derivate steht, die bzw. das in der Sequenz von Aconitase fehlt, und n und p ganze Zahlen zwischen 0 und 4 sind,
R₁ für die primäre Aminofunktion der N-terminalen Aminosäure steht, die frei oder durch eine Schutzgruppe substituiert ist, die aus einer Acetylgruppe, einer Benzoylgruppe, einer Tosylgruppe oder einer Benzyloxycarbonylgruppe ausgewählt sein kann,
R₂ für die Hydroxylgruppe der Carboxylfunktion der C-terminalen Aminosäure steht, die frei oder durch eine Schutzgruppe substituiert ist, die aus einer C₁- bis C₂₀-Alkylkette oder einer NH2-, NHY- oder NYY-Gruppe, wobei Y für eine C₁- bis C₄-Alkylkette steht, ausgewählt sein kann,
**dadurch gekennzeichnet, dass** das besagte Peptid der allgemeinen Formel (I) die folgende Sequenz aufweist:
(SEQ ID Nr. 1) Ser-Cys-Ile-Asn-Thr,
(SEQ ID Nr. 2) Ser-Cys-Ile-Asn-Thr-NH₂,
(SEQ ID Nr. 5) Ser-Cys-Ile-Asn-Ser.

2. Zusammensetzung nach Anspruch 1, **dadurch gekennzeichnet, dass** das besagte Peptid der allgemeinen Formel (I) der Sequenz SEQ ID Nr. 1 entspricht.

3. Zusammensetzung nach Anspruch 1, **dadurch gekennzeichnet, dass** das besagte Peptid der allgemeinen Formel (I) der Sequenz SEQ ID Nr. 2 entspricht.

4. Zusammensetzung nach einem der vorhergehenden Ansprüche, **dadurch gekennzeichnet, dass** das besagte Peptid der allgemeinen Formel (I) über mindestens eine funktionelle Gruppe verfügt, die durch eine Schutzgruppe geschützt ist, wobei diese Schutzgruppe entweder eine Acylierung oder eine Acetylierung des aminoterminalen Endes oder eine Amidierung oder eine Veresterung des carboxyterminalen Endes oder beides ist.

5. Zusammensetzung nach einem der vorhergehenden Ansprüche, **dadurch gekennzeichnet, dass** das besagte Peptid der allgemeinen Formel (I) in der Zusammensetzung in einer Konzentration zwischen 0,0005 und 500 ppm vorliegt.

6. Zusammensetzung nach Anspruch 5, **dadurch gekennzeichnet, dass** das besagte Peptid der allgemeinen Formel (I) in der Zusammensetzung in einer Konzentration zwischen 0,01 und 5 ppm vorliegt.

7. Zusammensetzung nach einem der vorhergehenden Ansprüche, **dadurch gekennzeichnet, dass** das besagte Peptid der allgemeinen Formel (I) zuvor in einem oder mehreren kosmetisch oder pharmazeutisch unbedenklichen Lösungsmitteln solubilisiert wird, das bzw. die aus Wasser, Glycerin, Ethanol, Propylenglykol, Butylenglykol, Dipropylenglykol, ethoxylierten oder propoxylierten Diglykolen, cyclischen Polyolen, Vaseline, einem pflanzlichen Öl oder jeglichem Gemisch dieser Lösungsmittel ausgewählt ist bzw. sind.

8. Zusammensetzung nach einem der vorhergehenden Ansprüche, **dadurch gekennzeichnet, dass** sie in einer Form vorliegt, die an die topische Anwendung angepasst ist, die ein kosmetisch oder dermatologisch unbedenkliches Medium umfasst.

9. Zusammensetzung nach einem der vorhergehenden Ansprüche, **dadurch gekennzeichnet, dass** sie außerdem mindestens einen weiteren Wirkstoff enthält, der die Wirkung des Peptids der allgemeinen Formel (I) begünstigt und aus Vitamin C, Vitamin E, Coenzym Q10, polyphenolischen Pflanzenextrakten, dem Peptid mit der Sequenz Gly-Pro-Gln-Gly-Pro-Gln, das unter dem Namen COLLAXYL® von Vincience vertrieben wird, oder dem *Artemia*-Extrakt, der unter dem Namen GP4G® von Vincience vertrieben wird, ausgewählt ist.

10. Wirkstoff, wie in einem der Ansprüche 1 bis 7 definiert, zu dessen Verwendung in einer Zusammensetzung, die dazu vorgesehen ist, die Haut und die Phanere gegen UV-Strahlen zu schützen.

11. Wirkstoff, wie in einem der Ansprüche 1 bis 7 definiert, zu dessen Verwendung in einer Zusammensetzung, die dazu vorgesehen ist, Schäden zu verhindern oder zu behandeln, die der Haut und den Phaneren durch freie Radikale verursacht werden.

12. Verwendung einer wirksamen Menge eines Wirkstoffs, wie in einem der Ansprüche 1 bis 7 definiert, in einer kosmetischen Zusammensetzung, **dadurch gekennzeichnet, dass** die Zusammensetzung dazu vorgesehen ist, Hautzeichen einer Alterung und/oder Lichtalterung zu verhindern oder zu behandeln.

13. Wirkstoff, wie in einem der Ansprüche 1 bis 7 definiert, zu dessen Verwendung in einer pharmazeutischen Zusammensetzung, die dazu vorgesehen ist, mitochondriale Dysfunktionen zu verhindern oder zu bekämpfen, die für bestimmte neuromuskuläre oder kardiale Degenerationen, Typ-II-Diabetes oder auch bestimmte Pathologien der Alterung verantwortlich sind.

## Claims

1. Cosmetic or pharmaceutical composition containing, in a physiologically acceptable medium, as an active ingredient protecting mitochondria, a peptide capable of activating aconitase, comprising 3 to 14 amino acid residues, and wherein the sequence corresponds to the general formula (I):
R₁-(AA)ₙ-X₁-X₂-X₃-X₄-X₅-X₆-(AA)ₚ-R₂
wherein
X₁ is serine,
X₂ is cysteine,
X₃ is theronine or isoleucine,
X₄ is glutamine, asparagine or no amino acid,
X₅ is threonine, serine or no amino acid,
X₆ is isoleucine, glutamine or no amino acid,
AA represents any amino acid or derivative thereof,
absent from the sequence of aconitase, and n and p are integers between 0 and 4,
R₁ represents the primary amine function of the N-terminal amino acid, free or substituted by a protecting group that may be chosen from an acetyl group, a benzoyl group, a tosyl group or a benzyloxycarbonyl group,
R₂ represents the hydroxyl group of the carboxyl function of the C-terminal amino acid, free or substituted by a protecting group that may be chosen from a C₁ to C₂₀ alkyl chain, or an NH2, NHY or NYY group where Y represents a C₁ to C₄ alkyl chain, **characterised in that** said peptide having general formula (I) is the sequence:
(SEQ ID NO. 1) Ser-Cys-Ile-Asn-Thr
(SEQ ID No. 2) Ser-Cys-Ile-Asn-Thr-NH₂
(SEQ ID No. 5) Ser-Cys-Ile-Asn-Ser.

2. Composition according to claim 1, **characterised in that** said peptide having general formula (I) corresponds to the sequence SEQ ID No. 1.

3. Composition according to claim 1, **characterised in that** said peptide having general formula (I) corresponds to the sequence SEQ ID No. 2.

4. Composition according to any of the above claims, **characterised in that** said peptide having general formula (I) has at least one functional group protected by a protecting group, this protecting group being either an acylation or an acetylation of the amino-terminus, or an amidation or an esterification of the carboxy-terminus, or both.

5. Composition according to any of the above claims, **characterised in that** the peptide having general formula (I) is present in the composition at a concentration between 0.0005 and 500 ppm.

6. Composition according to claim 5, **characterised in that** said peptide having general formula (I) is present in the composition at a concentration between 0.01 and 5 ppm.

7. Composition according to any of the above claims, **characterised in that** said peptide having general formula (I) is previously solubilised in one or a plurality of cosmetically or pharmaceutically acceptable solvents, such as water, glycerol, ethanol, propylene glycol, butylene glycol, dipropylene glycol, ethoxylated or propoxylated diglycols, cyclic polyols, petroleum jelly, vegetable oil or any mixture of these solvents.

8. Composition according to any of the above claims, **characterised in that** it is presented in a form suitable for topical application comprising a cosmetically or dermatologically acceptable medium.

9. Composition according to any of the above claims, **characterised in that** it further contains at least one further active ingredient promoting the action of said peptide having general formula (I) chosen from vitamin C, vitamin E, coenzyme Q10, plant polyphenolic extracts, the peptide having the sequence Gly-Pro-Gln-Gly-Pro-Gln marketed under the name Collaxyl® by Vincience, and *Artemia* extract marketed under the name GP4G® by Vincience.

10. Active ingredient, as defined in any of claims 1 to 7, for the use thereof in a composition for protecting the skin and skin appendages from UV rays.

11. Active ingredient, as defined in any of claims 1 to 7, for the use thereof in a composition for preventing or treating damage caused to the skin and skin appendages by free radicals.

12. Use in a cosmetic composition, of an effective quantity of active ingredient, as defined in any of claims 1 to 7, **characterised in that** the composition is for preventing or treating the skin signs of ageing and/or photoageing.

13. Active ingredient, as defined in any of claims 1 to 7, for the use thereof in a pharmaceutical composition for preventing or combating mitochondrial dysfunction responsible for certain forms of muscle or cardiac degeneration, type II diabetes or certain conditions associated with ageing.


### SEQUENCE LISTING

<110> Société d'Extraction des Principes Actifs (ISP VINCIENCE)
<120> COMPOSITION PHARMACEUTIQUE ET/OU COSMETIQUE CONTENANT DES PRINCIPES ACTIFS ACTIVATEURS DE L'ACONITASE
<130> Bv PCT 07-81
<150> FR 0703053
   <151> 2007-04-27
<160> 7
<170> PatentIn version 3.3
<210> 1
   <211> 5
   <212> PRT
   <213> Artificial
<220>
   <223> Synthetic Peptide
<400> 1
   Ser Cys Ile Asn Thr
   1               5
<210> 2
   <211> 5
   <212> PRT
   <213> Artificial
<220>
   <223> Synthetic Peptide
<220>
   <221> MOD_RES
   <222> (5)..(5)
   <223> AMIDATION
<400> 2
   Ser Cys Ile Asn Thr
   1               5
<210> 3
   <211> 5
   <212> PRT
   <213> Artificial
<220>
   <223> Synthetic Peptide
<400> 3
   Ser Cys Thr Asn Thr
   1               5
<210> 4
   <211> 5
   <212> PRT
   <213> Artificial
<220>
   <223> Synthetic Peptide
<220>
   <221> MOD_RES
   <222> (5)..(5)
   <223> AMIDATION
<400> 4
   Ser Cys Thr Asn Thr
   1               5
<210> 5
   <211> 5
   <212> PRT
   <213> Artificial
<220>
   <223> Synthetic Peptide
<400> 5
   Ser Cys Ile Asn Ser
   1               5
<210> 6
   <211> 4
   <212> PRT
   <213> Artificial
<220>
   <223> Synthetic Peptide
<400> 6
   Ser Cys Ile Gln
   1
<210> 7
   <211> 3
   <212> PRT
   <213> Artificial
<220>
   <223> Synthetic Peptide
<400> 7
   Ser Cys Thr
   1